(19) Europäisches Patentamt
European Patent Office
Office européen des brevets

(11) Numéro de publication: **0 088 027**
**B1**

# (12) FASCICULE DE BREVET EUROPEEN

(45) Date de publication du fascicule du brevet: **12.03.86**

(21) Numéro de dépôt: **83400429.3**

(22) Date de dépôt: **02.03.83**

(51) Int. Cl.⁴: **C 07 C 43/23**, C 07 C 49/84, C 07 C 69/96, C 07 C 147/06, C 08 F 16/32, C 08 G 18/32, C 08 G 77/42, C 07 C 41/03

(54) **Procédé de préparation de dérivés de polyphénols, nouveaux dérivés obtenus par ce procédé, leur utilisation pour la préparation de polymères et de copolymères et les copolymères ainsi obtenus.**

(30) Priorité: **03.03.82 FR 8203522**

(43) Date de publication de la demande: **07.09.83 Bulletin 83/36**

(45) Mention de la délivrance du brevet: **12.03.86 Bulletin 86/11**

(84) Etats contractants désignés: **DE GB IT NL**

(56) Documents cités:
**EP - A - 0 022 073**
**FR - A - 2 080 272**
**FR - A - 2 483 441**
**GB - A - 768 748**
**US - A - 4 042 566**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Laval, François, 2, Allée d'Alexandrie, F-77420 Champs-sur-Marne (FR)**
Inventeur: **Madec, Pierre Jean, 23, rue de la Manufacture, F-45000 Orléans (FR)**
Inventeur: **Maréchal, Ernest, 3, rue de Bretagne, F-75003 Paris (FR)**

(74) Mandataire: **Mongrédien, André et al, c/o BREVATOME 25, rue de Ponthieu, F-75008 Paris (FR)**

**EP 0 088 027 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé de préparation de dérivés de polyphénols, les dérivés obtenus par ce procédé, leur utilisation pour la préparation de polymères et copolymères éventuellement réticulés, et les copolymères ainsi obtenus.

De façon plus précise, elle concerne la préparation de dérivés de polyphénol comportant à chaque extrémité au moins deux fonctions réactives de nature différente, constituées respectivement par une fonction hydroxyle et par une fonction allylique.

De tels dérivés présentent un grand intérêt pour la fabrication de polymères et de copolymères statistiques, séquencés, greffés, réticulés ou non, utilisables par exemple pour la réalisation d'adhésifs, de plastiques renforcés ou de pièces devant résister à haute température.

Selon l'invention, le procédé de préparation d'un dérivé de polyphénol comportant au moins deux fonctions réactives de nature différente, à chacune de ses extrémités, se caractérise en ce qu'il consiste à faire réagir l'allyloxy-1 époxy-2,3 propane de formule:

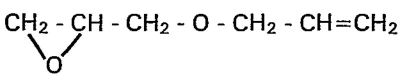

avec un polyphénol choisi parmi les composés suivants:
les polyphénols répondant à la formule:

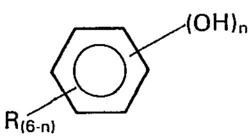

(I)

dans laquelle R représente un atome d'hydrogène, un atome d'halogène, un radical alkyle en $C_1$ à $C_5$ ou un radical alcoxy en $C_1$ à $C_5$ et n est un nombre entier de 3 à 6.

Selon un second mode de mise en œuvre les polyphénols répondant à la formule:

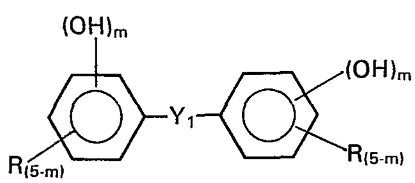

(II)

dans laquelle R représente un atome d'hydrogène, un atome d'halogène, un radical alkyle en $C_1$ à $C_5$ ou un radical alcoxy en $C_1$ à $C_5$, $Y_1$ représente

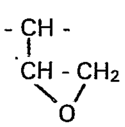 ou le groupe 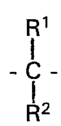

où $R^1$ et $R^2$ qui peuvent être identiques ou différents, représentent un atome d'halogène, un radical alcoxy en $C_1$ à $C_4$, un radical cycloalkyle en $C_3$ à $C_6$ ou le groupe 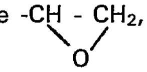, et m est un nombre entier de 1 à 5,

les polyphénols répondant à la formule:

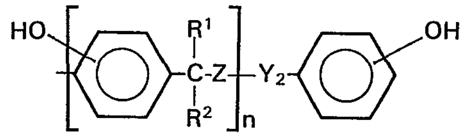

(III)

dans laquelle $R^1$ et $R^2$ qui peuvent être identiques ou différents, représentent l'hydrogène, un atome d'halogène, un radical alkyle en $C_1$ à $C_4$, un radical alcoxy en $C_1$ à $C_4$, un radical cycloalkyle en $C_3$ à $C_6$ ou le groupement 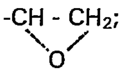;

Z représente le groupe

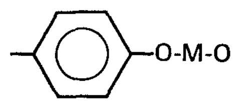

(A)

avec M représentant -CO ou

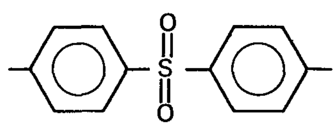

(B)

$Y_2$ représente le radical

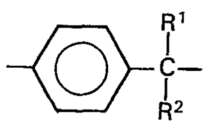

(C)

ou le radical

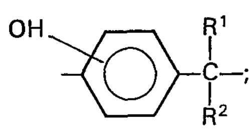

; et (D)

n est un nombre entier supérieur ou égal à 1;

les polyphénols de formule:

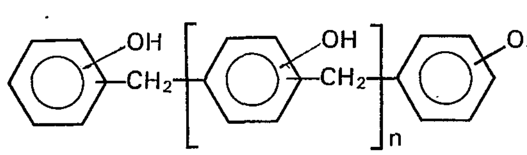

(IV)

dans laquelle n est un nombre entier supérieur ou égal à 1, et les polyphénols choisis dans le groupe comprenant le 1,1-bis(4-hydroxyphénylcyclohexane) (bisphénol C), et le phloroglucinol, en présence d'un catalyseur basique constitué soit par une amine tertiaire, soit par un hydroxyde alcalin.

Grâce à la mise en œuvre de cette réaction en présence d'un catalyseur constitué par une amine ou un hydroxyde alcalin, on peut obtenir rapidement et quantitativement une modification de la fonction phénol en une fonction double: une fonction allyle et une fonction hydroxyle.

De préférence, le polyphénol est un dérivé du bisphénol A tel qu'une polysulfone α,ω-diphénol ou un polycarbonate α,ω-diphénol.

A titre d'exemples d'amines tertiaires susceptibles d'être utilisées comme catalyseur dans le procédé de l'invention on peut citer la diméthyldodécylamine et la triéthanolamine.

De préférence, on réalise la réaction en l'absence de solvant à une température suffisante pour obtenir la solubilité du polyphénol dans l'allyloxy-1 époxy-2,3 propane et on opère à une température supérieure à 80°C, généralement à une température de 120°C.

Toutefois, on peut réaliser cette réaction en présence d'un solvant inerte tel que le chlorobenzène ou le diméthyl éther du diéthylène glycol (Diglyme).

L'invention a également pour objet les nouveaux dérivés de polyphénol obtenus par le procédé de l'invention.

Selon un premier mode de réalisation de l'invention ces dérivés répondent à la formule:

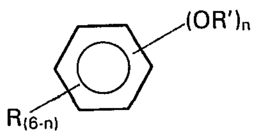

(V)

dans laquelle R représente un atome d'hydrogène, un atome d'halogène, un radical alkyle en $C_1$ à $C_5$ ou un radical alcoxy en $C_1$ à $C_5$, n représente un nombre entier de 3 à 6 et R' représente

$$-CH_2-\underset{OH}{CH}-CH_2-O-CH_2-CH=CH_2$$

Selon un deuxième mode de réalisation de l'invention ces dérivés répondent à la formule:

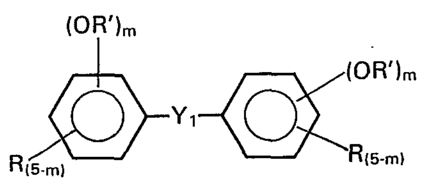

(VI)

dans laquelle R' représente

$$-CH_2-\underset{OH}{CH}-CH_2-O-CH_2-CH=CH_2,$$

R représente un atome d'hydrogène, un atome d'halogène, un radical alkyle en $C_1$ à $C_5$ ou un radical alcoxy en $C_1$ à $C_5$;

$Y_1$ représente

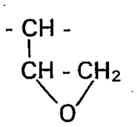 ou le groupe 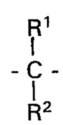

avec $R^1$ et $R^2$ qui peuvent être identiques ou différents, représentant un atome d'halogène, un radical alcoxy en $C_1$ à $C_4$, un radical cycloalkyle en $C_3$ à $C_6$ ou le groupement 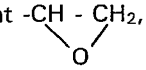, et m est un nombre entier de 1 à 5.

Selon un troisième mode de réalisation de l'invention ces dérivés répondent à la formule suivante:

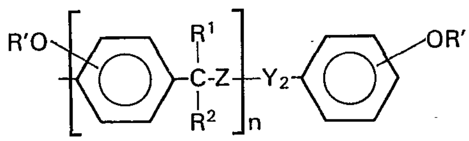

(VII)

dans laquelle R' représente

$$-CH_2-\underset{OH}{CH}-CH_2-O-CH_2-CH=CH_2$$

$R^1$ et $R^2$ qui peuvent être identiques ou différents, représentent un atome d'hydrogène, un atome d'halogène, un radical alkyle en $C_1$ à $C_4$, un radical alcoxy en $C_1$ à $C_4$, un radical cycloalkyle en $C_3$ à $C_6$ ou le groupement 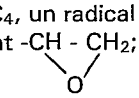;

Z représente le groupe

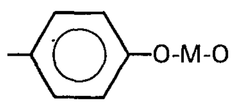

(A)

avec M représentant CO ou

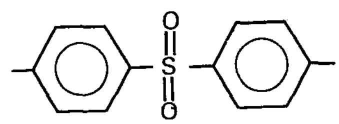

(B)

$Y_2$ représente le radical

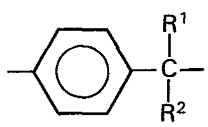

(C)

ou le radical

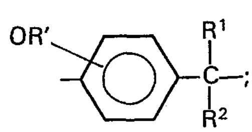; et

(D)

et n est un nombre entier égal ou supérieur à 1.

A titre d'exemple de tels dérivés on peut citer:

- le dérivé répondant à la formule:

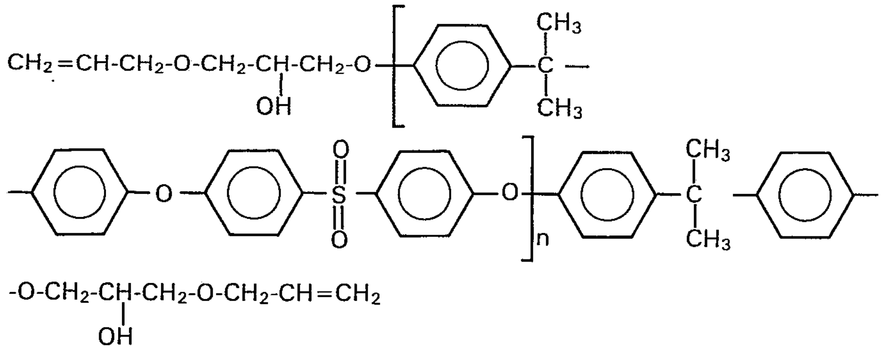

(X)

avec n étant un nombre entier égal ou supérieur à 1, qui peut être obtenu par réaction d'une polysulfone α,ω-diphénol de formule:

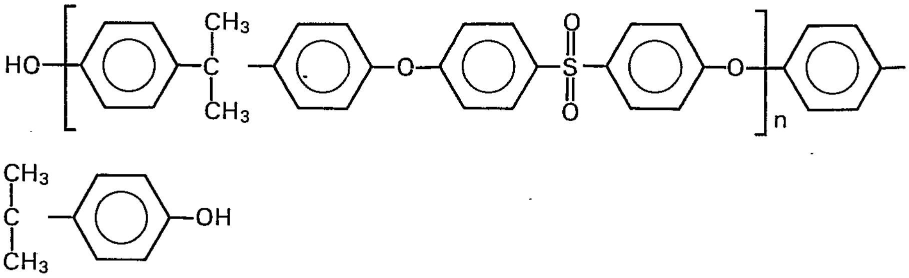

(XI)

dans laquelle n est un nombre entier supérieur ou égal à 1, avec l'allyloxy-1 époxy-2,3 propane en présence d'une amine tertiaire ou d'un hydroxyde alcalin; et

- le dérivé répondant à la formule:

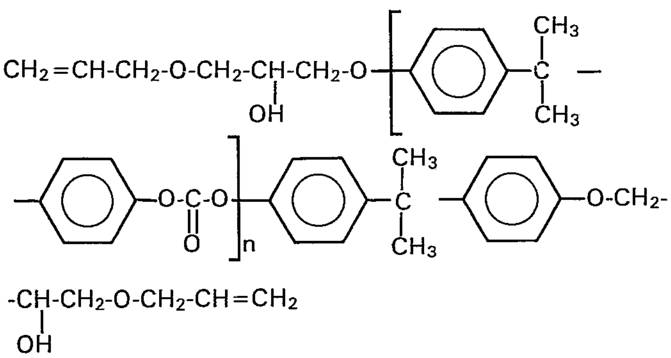

(XII)

dans laquelle n est un nombre entier égal ou supérieur à 1, qui peut être obtenu en faisant réagir un polycarbonate α,ω-diphénol de formule:

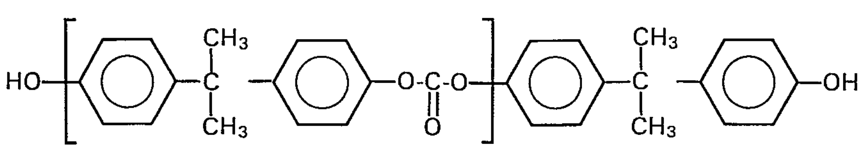

(XIII)

dans laquelle n est un nombre entier égal ou supérieur à 1 avec l'allyloxy-1 époxy-2,3 propane en présence d'un catalyseur constitué par une amine tertiaire ou un hydroxyde alcalin.

Les polycarbonates de formule (XIII) utilisés comme produits de départ pour la synthèse de dérivés de polyphénol selon l'invention sont obtenus par réaction du bis-phénol A avec le $COCl_2$ par des procédés classiques. De même, les polysulfones de formule (XI) utilisés comme produits de départ dans la synthèse des dérivés de polyphénol de l'invention pouvent être obtenus par réaction du bis-phénol A avec le dichlorodiphénylsulfone par des procédés classiques.

Selon un quatrième mode de réalisation de l'invention, le dérivé de polyphénol répond à la formule suivante:

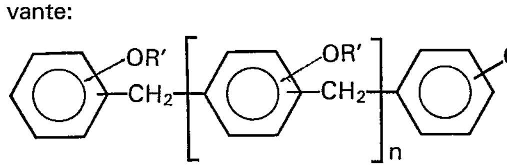

(XIV)

avec R' représentant

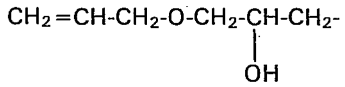

et n représentant un nombre entier supérieur ou égal à 1.

Un tel dérivé peut être obtenu en faisant réagir un résol de formule:

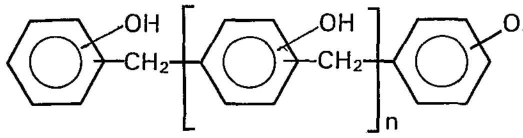

(IV)

avec l'allyloxy-1 époxy-2,3 propane en présence d'un catalyseur constitué par une amine tertiaire.

Les résols de formule (VIII) utilisés comme produits de départ sont obtenus par réaction d'un phénol avec un formol à chaud en présence de catalyseur alcalin selon des procédés connus.

Les dérivés de polyphénol de l'invention présentent un grand intérêt en raison de la présence simultanée de deux types différents de fonctions réactives à chaque extrémité de la chaîne macromoléculaire et éventuellement des groupements latéraux, c'est-à-dire d'une première fonction réactive constituée par le groupement allylique et d'une deuxième fonction réactive constituée par le groupement hydroxyle secondaire.

De tels dérivés peuvent ainsi être utilisés pour réaliser la synthèse de copolymères séquencés et de matériaux réticulés par l'intermédiaire de l'une ou l'autre de ces deux fonctions réactives.

On rappelle que des copolymères séquencés sont constitués d'enchaînements alternés de chaînons polymères de natures chimiques différentes A et B, les diverses structures possibles étant les suivantes: BAB, ABA, et $(AB)_n$.

Ainsi, avec ces structures on peut allier les propriétés de deux dérivés différents dans un seul et même matériau et alterner, par exemple, des séquences rigides avec des séquences à caractère élastomère. Ceci peut être obtenu avec les dérivés de polyphénol obtenus par réaction d'un polyphénol avec l'allyloxy-1 époxy-2,3 propane, à partir de l'une ou l'autre des deux fonctions réactives présentes à chaque extrémité de la molécule et l'on peut ensuite utiliser la seconde fonction réactive qui n'a pas servi pour la fabrication du copolymère séquencé pour d'autres réactions par exemple, pous des réactions de réticulation.

En particulier, on peut utiliser la fonction allylique pour effectuer une homopolymérisation ou une copolymérisation avec des groupements éthyléniquement insaturés, en présence d'un catalyseur radicalaire ou ionique adéquat.

Ainsi, on peut obtenir un réseau tridimensionnel et la réactivité du catalyseur peut être choisie de façon telle que la réaction se produise à température suffisamment élevée de telle sorte qu'au dessous de cette température, le polymère puisse être encore mis en œuvre.

On peut encore utiliser la fonction allyle pour effectuer des réactions d'addition avec d'autres fonctions par exemple avec les silanes-(Si-H) ou thiol-(S-H).

A titre d'exemple, l'utilisation de cette réaction est intéressante pour la préparation de copolymères séquencés polysulfones-polysiloxanes BAB, ABA ou $(AB)_n$, la séquence polysulfone A constituant la phase rigide et la séquence polysiloxane B constituant la phase élastomère du copolymère.

Dans ce cas, le dérivé B participant à la réaction de copolymérisation peut être un polysiloxane comportant à ses extrémités des fonctions silanes, par exemple le polydiméthylsiloxane alpha, oméga-dihydrogéno de formule:

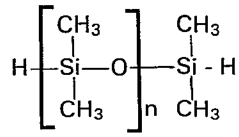

(XV)

avec n supérieur ou égal à 1.

La réaction de polycondensation est la suivante:

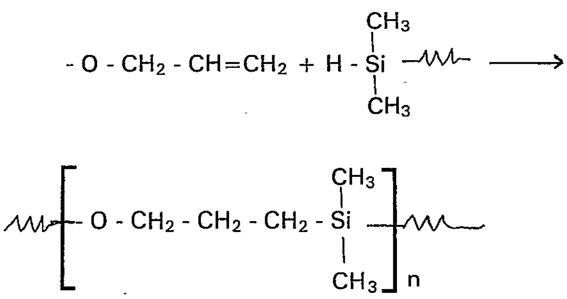

Cette réaction peut être effectuée facilement.

De tels polymères séquencés peuvent présenter une thermostabilité élevée et une viscosité relativement faible, et il est possible de régler cette viscosité en utilisant des dérivés de longueurs variées. Lorsque les copolymères ont une faible viscosité, ceux-ci peuvent être mis en œuvre aisément, par exemple par injection.

Selon l'invention, on peut utiliser les groupements hydroxyle des dérivés de polyphénol ou du copolymère séquencé pour former par réaction par exemple avec des acides dicarboxyliques ou des dérivés de diacides dicarboxyliques tels que les anhydrides, un réseau tridimensionnel.

On peut aussi faire réagir la fonction hydroxyle avec un polyisocyanate tel que le toluène diisocyanate (TDI), l'hexaméthylène diisocyanate (HMDI) et le diphénylméthane diisocyanate, selon le schéma réactionnel suivant:

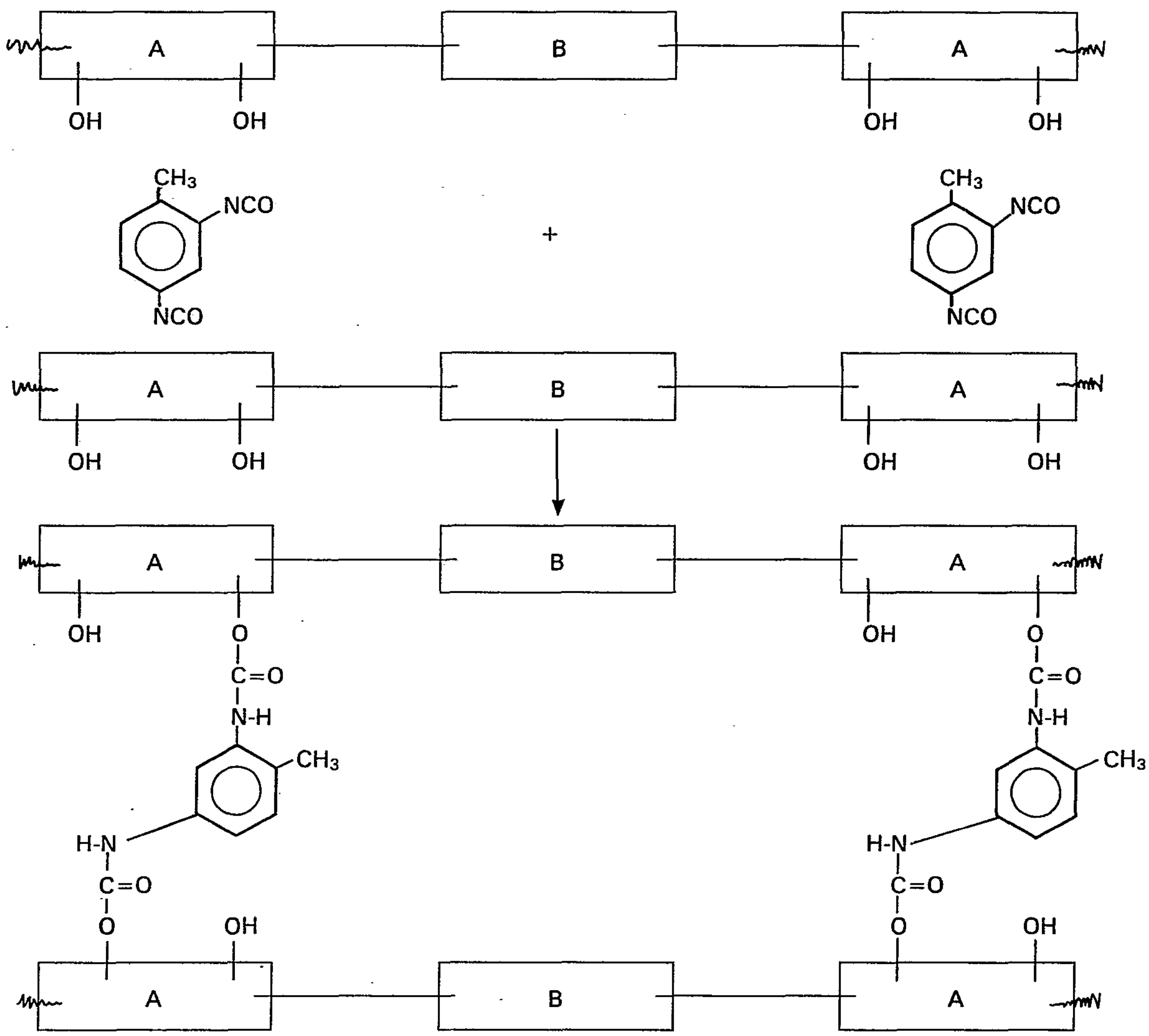

D'autres caractéristiques et avantages de l'invention apparaitront mieux à la lecture des exemples suivants donnés bien entendu à titre illustratif et non limitatif.

*Exemple 1*

On mélange dans un réacteur muni d'un système d'agitation et d'un système permettant un balayage d'azote, une mole de 1,1-bis(4-hydroxyphénylcyclohexane) et 228 g d'allyloxy-1-époxy-2,3 propane (2 mol). On chauffe ensuite le mélange réactionnel jusqu'à 120°C. On introduit alors 10 g d'une amine tertiaire constituée par la N,N-diméthyldodécylamine et on maintient la température à 120°C pendant deux heures. On identifie par résonance magnétique nucléaire, spectrométrie infrarouge et chromatographie par perméation de gel, le produit obtenu qui correspond bien au produit de la réaction du 1,1-bis(4--hydroxyphénylcyclohexane) avec l'allyloxy-1-époxy--2,3 propane. On prépare selon le même mode opératoire le dérivé de phloroglucinol que l'on identifie également par résonance magnétique nucléaire, spectrométrie infrarouge et chromatographie par perméation de gel.

*Exemple 2*

Cet exemple concerne la préparation d'un dérivé répondant à la formule X dans laquelle n est égal à 4.

On mélange dans un réacteur muni d'un système d'agitation et d'un système permettant un balayage à l'azote, 20 g de polysulfone alpha, oméga diphénol de formule (XI) avec n=4 (1/100 de mole) et 23 g d'allyloxy-1 époxy-2,3 propane (0,13 mole), ce qui correspond à un excès. On porte ensuite l'ensemble à une température de 120°C, et la solubilisation de la polysulfone dans l'allyglycidyl éther apparaît pendant la période de préchauffage. On ajoute ensuite 0,2 g de N,N-diméthyldodécylamine et on poursuit la réaction à la température de 120°C pendant 3 h. Le polymère obtenu est purifié par précipitation dans l'hexane après dissolution dans du chlorure de méthylène. On élimine ainsi l'excès d'allyloxy-1 époxy-2,3 propane et son homopolymère éventuel. On obtient ainsi 20 g d'une poudre beige (rendement supérieur à 95%) qui comprend un mélange de l'oligomère bifonctionnel de formule X dans laquelle n est égal à 4 et essentiellement des oligomères polyfonctionnels de fonctionnalité supérieure à 2 résultant de la réaction des fonctions hydroxyle de la polysulfone avec l'allyloxy-1 époxy-2,3 propane.

*Exemple 3*

On mélange dans un réacteur muni d'un système d'agitation et d'un système permettant un balayage d'azote, 20 g (1/100 de mole) de la polysulfone de l'exemple 3 et 2,3 g d'allyloxy-1 époxy-2,3 propane (2/100 de mole) à 50 ml de chlorobenzène. On élève la température jusqu'à 120°C et on ajoute 0,1 g de N,N diméthyldodécylamine, puis on poursuit la réaction pendant 24 heures à cette température. On élimine ensuite le solvant sous vide et on sèche le produit obtenu à 80°C sous (13,5 Pa) pendant une heure. Le rendement de la réaction est supérieur à 95% et le produit obtenu correspond à l'oligomère bifonctionnel de formule X dans laquelle n est égal à 4.

*Exemple 4*

Cet exemple concerne la préparation d'un oligomère répondant à la formule X dans laquelle n est égal à 4.

On mélange dans un réacteur muni d'un système d'agitation et d'un système permettant un balayage à l'azote, 20 g de polysulfone alpha, oméga diphénol de formule (XI) avec n=4 (1/100 de mole), 23 g d'allyloxy-1 époxy-2,3 propane et 20 g de diméthyléther du diéthylène glycol (diglyme). On porte ensuite l'ensemble à une température de 120°C et la solubilisation de la polysulfone dans le mélange allylglycidyl éther-diglyme apparaît pendant la période de préchauffage. On ajoute ensuite 0,2 g de N,N diméthyldodécylamine et on poursuit la réaction à la température de 120°C pendant 3 h. On purifie le polymère par précipitation dans l'hexane après dissolution dans du chlorure de méthylène pour éliminer l'excés de diglyme. On obtient ainsi 20 g d'une poudre beige (rendement supérieur à 95%) et on vérifie par analyse chimique, physicochimioque et spectroscopique qu'elle correspond bien à l'oligomère de formule X dans laquelle n est égal à 4.

*Exemple 5*

Cet exemple concerne la préparation d'un polymère séquencé à partir de (bis allyloxy-3 hydroxy-2 propanoxy-4 phényle) 2,2 propane de formule:

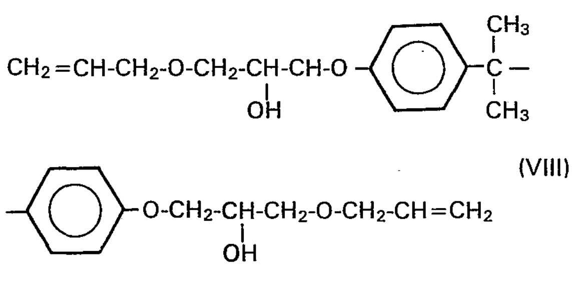

(VIII)

et de tétraméthyldisiloxane de formule XV avec n égal à 1.

On prépare le composé de formule VIII de la façon suivante.

On mélange dans un réacteur muni d'un système d'agitation et d'un système permettant un balayage d'azote, 228 g de bis-phénol A (1 mole) et 228 g d'allyloxy-1 époxy-2,3 propane (2 moles). On chauffe ensuite le mélange réactionnel, la solubilisation du bis-phénol A dans l'allyloxy-1 époxy-2,3 propane apparaît vers 70 à 80°C, et on poursuit le chauffage jusqu'à 120°C. On introduit alors 10 g d'une amine tertiaire constituée par la N,N-diméthyldodécylamine et on mantient la température à 120°C pendant 2 h. On obtient ainsi 466 g d'un produit visqueux jaunâtre mais transparent (rendement supérieur à 95%), et l'on vérifie par dosage chimique, par chromatographie par perméation de gel, par résonance magnétique nucléaire et par spectrométrie infrarouge que ce produit correspond bien à la formule VIII.

On mélange dans un réacteur muni d'un système d'agitation d'un condenseur et d'un système permettant un balayage d'azote 456 g (1 mole) du composé de formule VIII et 134 g (1 mole) de tétraméthyldisiloxane. On ajoute 1 ml d'une solution d'acide hexachloroplatinique dans du butanol tertiaire (solution à 1%). La température est montée progressivement à 70°C. Le milieu réactionnel opaque au début, en raison de l'incompatibilité des deux réactifs, devient translucide au bout de quelques minutes de réaction. La viscosité augmente rapidement. On obtient au bout d'une heure un produit visqueux jaunâtre transparent.

L'analyse par chromatographie par perméation de gel révèle une masse molaire moyenne en nombre supérieure à 10000.

La solubilisation de ce polymère est très rapide dans de nombreux solvants organiques.

*Exemple 6*

Cet exemple concerne la préparation d'un copolymère séquencé à partir du composé de formule VIII de l'exemple 5 et d'une huile polydiméthylsiloxane α,ω-dihydrogéno de formule XV ayant une masse moléculaire moyenne en nombre Mn ~ 1100.

On mélange dans un réacteur muni d'un système d'agitation et d'un système permettant un balayage d'azote 45,6 g (0,1 mole) du composé de formule VIII et 110 g (0,1 mole) de polydiméthylsiloxane α,ω-dihydrogéno. On ajoute 0,5 ml d'une solution d'acide hexachloroplatinique dans du butanol tertiaire (solution à 1%). La température est montée progressivement à 100°C. Comme dans l'exemple 6 le milieu réactionnel opaque au début en raison de l'incompatibilité des deux réactifs, devient translucide au bout de quelques minutes de réaction. La viscosité augmente rapidement. On obtient au bout d'une heure un produit visqueux jaunâtre mais transparent.

On obtient un polymère de masse molaire moyenne en nombre supérieure à 20000. Il est soluble dans de nombreux solvants organiques.

*Exemple 7*

Cet exemple concerne la préparation à partir du produit de formule VIII de l'exemple 5 d'un matériau réticulé par polymérisation radicalaire des doubles liaisons allyliques.

40,0 g du produit de formule VIII sont dégazés à 130°C dans un réacteur de 250 ml téfloné intérieurement, muni d'un système d'agitation, d'un thermomètre et d'une ampoule à brome. Après retour à la température ordinaire, un balayage d'azote est établi dans le réacteur et 4,0 g de perbenzoate de tertiobutyle sont introduits à l'aide de l'ampoule à brome. Après homogénéisation, on élève la température à 150° et on retire l'agitateur du mélange en cours de réaction. Cette température est maintenue pendant 24 heures.

On obtient ainsi un matériau insoluble et infusible de grande souplesse à la température ordinaire.

*Exemple 8*

Cette exemple concerne la préparation à partir du produit de formule VIII de l'exemple 5, d'un polyuréthanne par polycondensation d'un diisocyanate sur les groupements hydroxyles latéraux du produit de formule VIII (BPADA).

Dans un bécher de 100 ml, 46,5 g de BPDA (0,1 mole) sont mélangés à 17,4 g (0,1 mole) de toluène diisocyanate (TDI). La réaction est immédiate et fortement exothermique. On obtient ainsi un matériau thermoplastique dur et cassant dont le point de ramollissement se situe aux environs de 52 °C.

*Exemple 9*

Cet exemple concerne encore la préparation d'un polyuréthanne par condensation d'un diisocyanate sur les groupements hydroxyles latéraux du BPADA (produit de formule VIII).

Dans un bécher de 100 ml, 40,5 g de BPADA (0,1 mole) sont mélangés à 25,8 g (0,1 mole) de HMDI de formule $OCN-(CH_2)_6-NCO$. La température est élevée jusqu'à 110 °C. Au cours de cette élévation, la viscosité du mélange augmente progressivement.

On obtient un matériau thermoplastique souple.

*Exemple 10*

Cet exemple concerne la préparation d'un copolymère séquencé poly(sulfone-seq-siloxane).

On mélange dans un réacteur muni d'un système d'agitation, sous balayage d'azote, 200 g (0,1 mole) de la polysulfone modifiée obtenu dans l'exemple 4 avec 110 g du polydiméthyl siloxane utilisé dans l'exemple 6. On ajoute 0,5 ml d'une solution d'acide hexachloroplatinique dans du butanol tertiaire (solution à 1%), La température est montée progressivement à 100 °C. L'homogénéisation du milieu réactionnel intervient au bout de quelques minutes. La viscosité augmente rapidement.

On obtient un polymère de masse molaire moyenne en nombre supérieure à 20000.

**Revendications**

1. Procédé de préparation d'un dérivé de polyphénol comportant au moins deux fonctions réactives de nature différente à chacune de ses extrémités, caractérisé en ce qu'il consiste à faire réagir l'allyloxy-1 époxy-2,3 propane de formule:

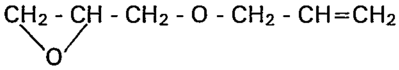

avec un polyphénol choisi parmi les composés suivants:

- le polyphénol répondant à la formule:

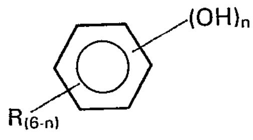

(I)

dans laquelle R représente un atome d'hydrogène, un atome d'halogène, un radical alkyle en $C_1$ à $C_5$ ou un radical alcoxy en $C_1$ à $C_5$ et n est un nombre entier de 3 à 6;

- le polyphénol répondant à la formule:

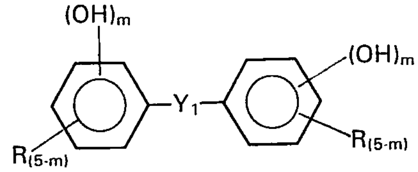

(II)

dans laquelle R représente un atome d'hydrogène, un atome d'halogène, un radical alkyle en $C_1$ à $C_5$ ou un radical alcoxy en $C_1$ à $C_5$, $Y_1$ représente

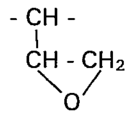 ou le groupe 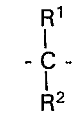

où $R^1$ et $R^2$ qui peuvent être identiques ou différents, représentent un atome d'halogène, un radical alcoxy en $C_1$ à $C_4$, un radical cycloalkyle en $C_3$ à $C_6$ ou le groupe 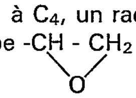

et m est un nombre entier de 1 à 5;

- le polyphénol répondant à la formule:

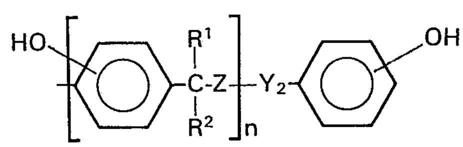

(III)

dans laquelle $R^1$ et $R^2$ qui peuvent être identiques ou différents, représentent l'hydrogène, un atome d'halogène, un radical alkyle en $C_1$ à $C_4$, un radical alcoxy en $C_1$ à $C_4$, un radical cycloalkyle en $C_3$ à $C_6$ ou le groupement 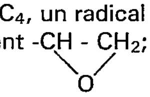;

Z représente le groupe

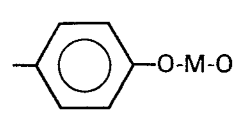

(A)

avec M représentant -CO ou

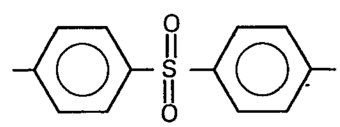

(B)

$Y_2$ représente le radical

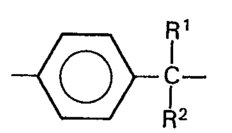

(C)

ou le radical

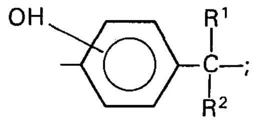 et (D)

n est un nombre entier supérieur ou égal à 1;

- le polyphénol répondant à la formule:

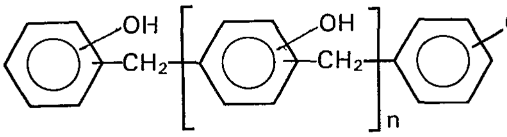 (IV)

dans laquelle n est un nombre entier égal ou supérieur à 1; et

- les polyphénols choisis dans le groupe comprenant le 1,1-bis(4-hydroxyphénylcyclohexane) bis-phénol C et le phloroglucinol,

en présence d'un catalyseur basique constitué soit par une amine tertiaire, soit par un hydroxyde alcalin.

2. Procédé selon la revendication 1, caractérisé en ce que l'amine tertiaire est la N,N-diméthyl dodécylamine ou la triéthanolamine.

3. Procédé selon l'une quelconque des revendications 1 et 2, caractérisé en ce que l'on réalise la réaction en l'absence de solvant à une température suffisante pour obtenir la solubilité du polyphénol dans l'allyloxy-1 époxy-2,3 propane.

4. Dérivé de polyphénol, caractérisé en ce qu'il répond à la formule suivante:

d 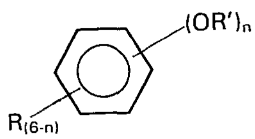 (V)

dans laquelle R représente un atome d'hydrogène, un atome d'halogène, un radical alkyle en $C_1$ à $C_5$ ou un radical alcoxy en $C_1$ à $C_5$, n représente un nombre entier de 3 à 6 et R' représente le groupement:

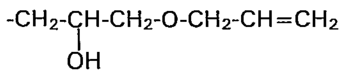

5. Dérivé de polyphénol, caractérisé en ce qu'il répond à la formule suivante:

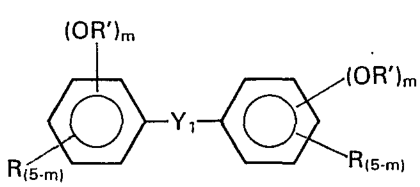 (VI)

dans laquelle R' représente:

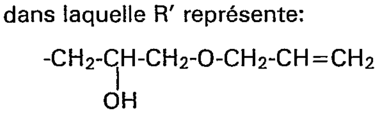

R représente2 un atome d'hydrogène, un atome d'halogène, un radical alkyle en $C_1$ à $C_5$ ou un radical alcoxy en $C_1$ à $C_5$;

$Y_1$ représente

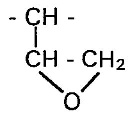 ou le groupe 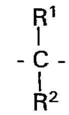

avec $R_1$ et $R_2$ qui peuvent être identiques ou différents, représentant un atome d'halogène, un radical alcoxy en $C_1$ à $C_4$, un radical cycloalkyle en $C_3$ à $C_6$ ou le groupement 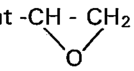

et m est un nombre entier de 1 à 5.

6. Dérivé de polyphénol, caractérisé en ce qu'il répond à la formule suivante:

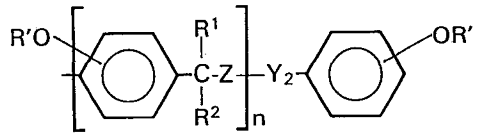 (VII)

dans laquelle R' représente:

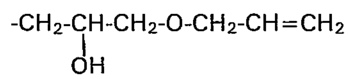

$R^1$ et $R^2$ qui peuvent être identiques ou différents, représentent un atome d'hydrogène, un atome d'halogène, un radical alkyle en $C_1$ à $C_4$, un radical alcoxy en $C_1$ à $C_4$, un radical cycloalkyle en $C_3$ à $C_6$ ou le groupement 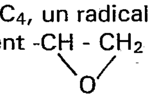 ;

Z représente le groupe

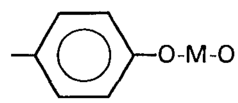 (A)

avec M représentant CO ou

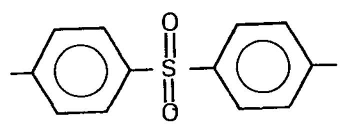 (B)

$Y_2$ représente le radical

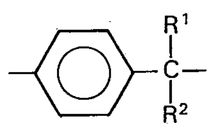 (C)

ou le radical

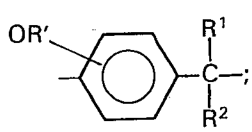 et (D)

n est un nombre entier égal ou supérieur à 1.

7. Dérivé de polyphénol selon la revendication 6, caractérisé en ce qu'il répond à la formule:

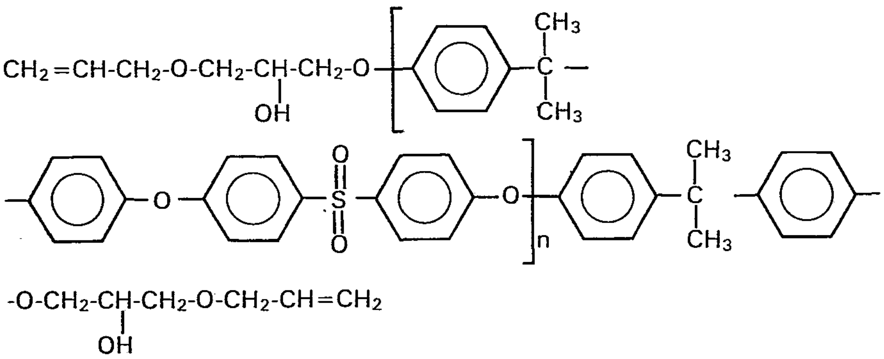

(X)

dans laquelle n est un nombre entier égal ou supérieur à 1.

8. Dérivé de polyphénol selon la revendication 6, caractérisé en ce qu'il répond à la formule:

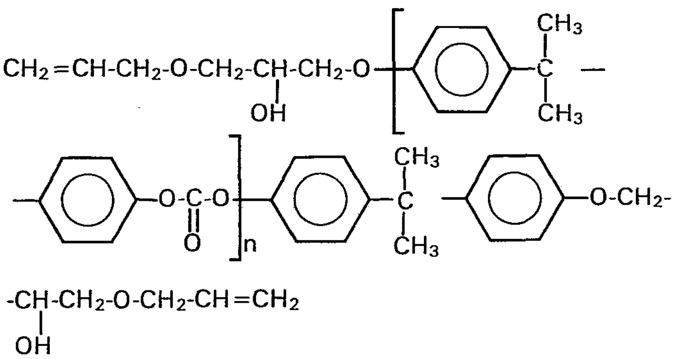

(XII)

dans laquelle n est un nombre entier supérieur ou égal à 1.

9. Dérivé de polyphénol selon la revendication 7, caractérisé en ce que n est égal à 4.

10. Dérivé de polyphénol, caractérisé en ce qu'il répond à la formule:

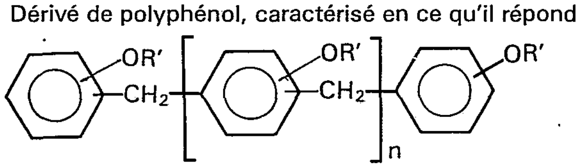

(XIV)

dans laquelle R' représente 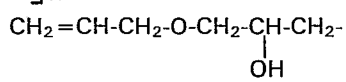

et n est un nombre entier supérieur ou égal à 1.

11. Copolymère séquencé d'un dérivé de polyphénol obtenu par réaction d'un polyphénol avec l'allyl-oxy-1 époxy-2,3-propane, et de polysiloxane de formule:

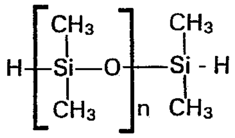

(XV)

dans laquelle n est supérieur ou égal à 1.

12. Copolymère selon la revendication 11, caractérisé en ce que le dérivé de polyphénol est le (bis allyloxy-3 hydroxy-2 propanoxy-4 phényle) 2,2 propane.

13. Copolymère selon la revendication 11, caractérisé en ce que le dérivé de polyphénol répond à la formule:

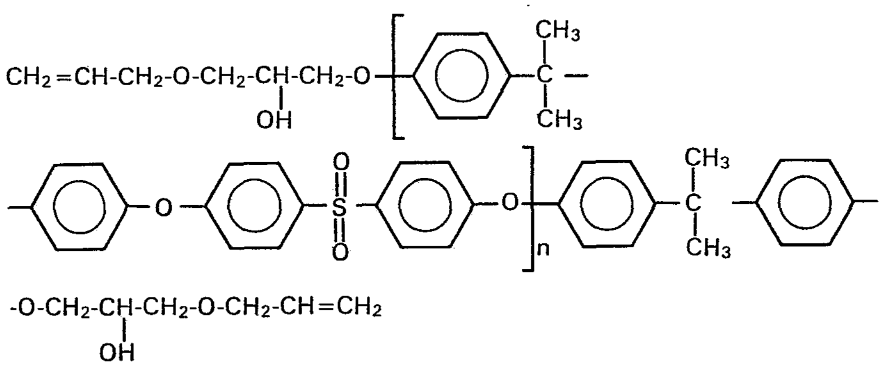

(X)

avec n étant un nombre entier supérieur ou égal à 1.

**Patentansprüche**

1. Verfahren zur Herstellung eines Polyphenolderivats, das an jedem seiner Enden mindestens zwei reaktive Funktionen unterschiedlicher Natur enthält, dadurch gekennzeichnet, dass man das 1-Allyloxy-2,3-epoxy-propan der Formel

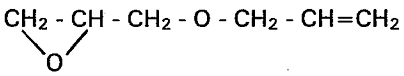

mit einem Polyphenol, das aus den folgenden Verbindungen ausgewählt wird:

- Polyphenol der Formel

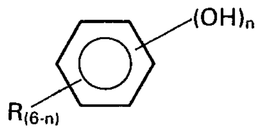

(I)

worin R ein Wasserstoffatom, ein Halogenatom, einen $C_1$-$C_5$-Alkylrest oder einen $C_1$-$C_5$-Alkoxyrest und n eine ganze Zahl von 3 bis 6 bedeuten;

- Polyphenol der Formel

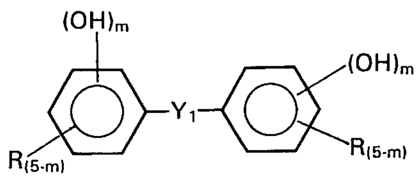

(II)

worin R ein Wasserstoffatom, ein Halogenatom, einen $C_1$-$C_5$-Alkylrest oder einen $C_1$-$C_5$-Alkoxyrest,

$Y_1$ die Gruppe

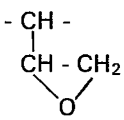 oder die Gruppe 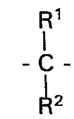

worin $R^1$ und $R^2$, die gleich oder verschieden sein können, jeweils ein Wasserstoffatom, einen $C_1$-$C_4$-Alkoxyrest, einen $C_3$-$C_6$-Cycloalkylrest oder die Gruppe 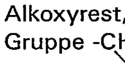 darstellen, und

m eine ganze Zahl von 1 bis 5 bedeuten;

- Polyphenol der Formel

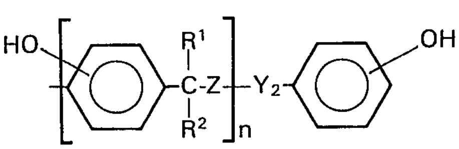

(III)

worin $R^1$ und $R^2$, die gleich oder verschieden sein können, jeweils ein Wasserstoffatom, ein Halogenatom, einen $C_1$-$C_4$-Alkylrest, einen $C_1$-$C_4$-Alkoxyrest, einen $C_3$-$C_6$-Cycloalkylrest oder die Gruppe 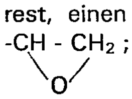 ;

Z die Gruppe

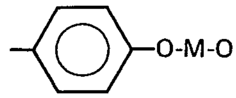

(A)

worin M -CO darstellt, oder die Gruppe

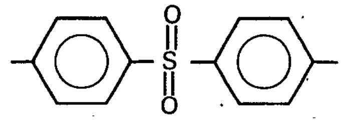

(B)

$Y_2$ den Rest

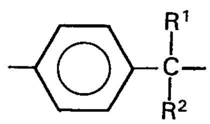

(C)

oder den Rest

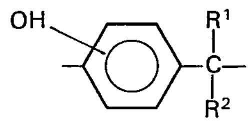

(D)

und

n eine ganze Zahl ≥ 1 bedeuten;

— Polyphenol der Formel

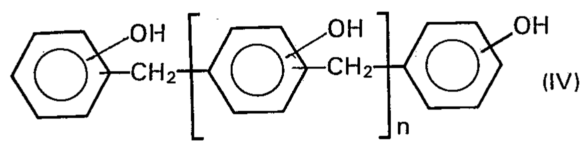

(IV)

worin n eine ganze Zahl ≥ 1 bedeutet; und

- Polyphenole, ausgewählt aus der Gruppe, die umfasst 1,1-Bis(4-hydroxyphenylcyclohexan) (Bisphenol C) und Phloroglucin,

in Gegenwart eines basischen Katalysators, der entweder aus einem tertiären Amin oder aus einem Alkalihydroxid besteht, reagieren lässt.

2. Verfahren nach Anspruch 1, dadurch gekennzeichnet, dass es sich bei dem tertiären Amin um N,N-Dimethyldodecylamin oder Triethanolamin handelt.

3. Verfahren nach einem der Ansprüche 1 und 2, dadurch gekennzeichnet, dass die Reaktion in Abwesenheit eines Lösungsmittels bei einer Temperatur durchgeführt wird, die ausreicht, um die Löslichkeit des Polyphenols in 1-Allyloxy-2,3-epoxy-propan zu erzielen.

4. Polyphenolderivat, dadurch gekennzeichnet, dass es der folgenden Formel entspricht:

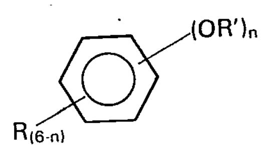

(V)

worin R ein Wasserstoffatom, ein Halogenatom, einen $C_1$-$C_5$-Alkylrest oder einen $C_1$-$C_5$-Alkoxy-

rest, n eine ganze Zahl von 3 bis 6 und R' die gruppe

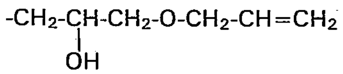

bedeuten.

5. Polyphenolderivat, dadurch gekennzeichnet, dass es der folgenden Formel entspricht:

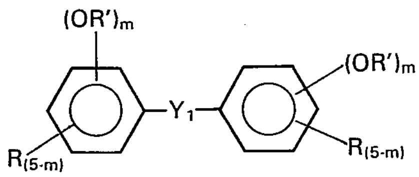

(VI)

worin R'

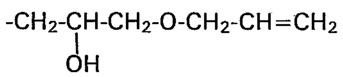

R ein Wasserstoffatom, ein Halogenatom, einen $C_1$-$C_5$-Alkylrest oder einen $C_1$-$C_5$-Alkoxyrest;

$Y_1$ die Gruppe

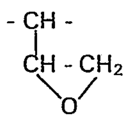 oder die Gruppe 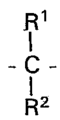

worin $R^1$ und $R^2$, die gleich oder verschieden sein können, jeweils ein Wasserstoffatom, einen $C_1$-$C_4$-Alkoxyrest, einen $C_3$-$C_6$-Cycloalkylrest oder die Gruppe 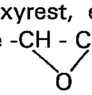 darstellen, und

m eine ganze Zahl von 1 bis 5 bedeuten.

6. Polyphenolderivat, dadurch gekennzeichnet, dass es der folgenden Formel entspricht:

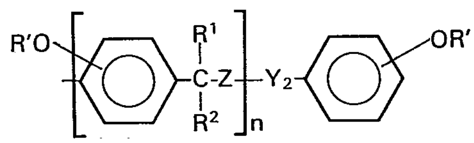

(VII)

worin R' die Gruppe

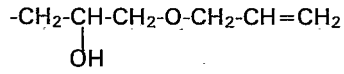

$R^1$ und $R^2$, die gleich oder verschieden sein können, ein Wasserstoffatom, ein Halogenatom, einen $C_1$-$C_4$-Alkylrest oder einen $C_1$-$C_4$-Alkoxyrest, einen $C_3$-$C_6$-Cycloalkylrest oder die Gruppe 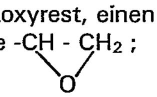

Z die Gruppe

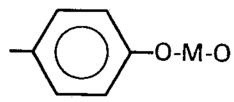

(A)

worin M für CO steht oder die Gruppe

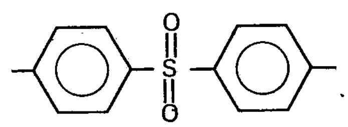

(B)

$Y_2$ den Rest

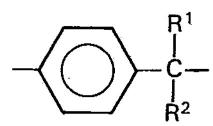

(C)

oder den Rest

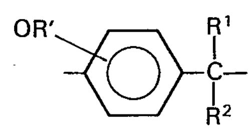

(D)

und

n eine ganze Zahl $\geq$ 1 bedeuten.

7. Polyphenolderivat nach Anspruch 6, dadurch gekennzeichnet, dass es der Formel entspricht:

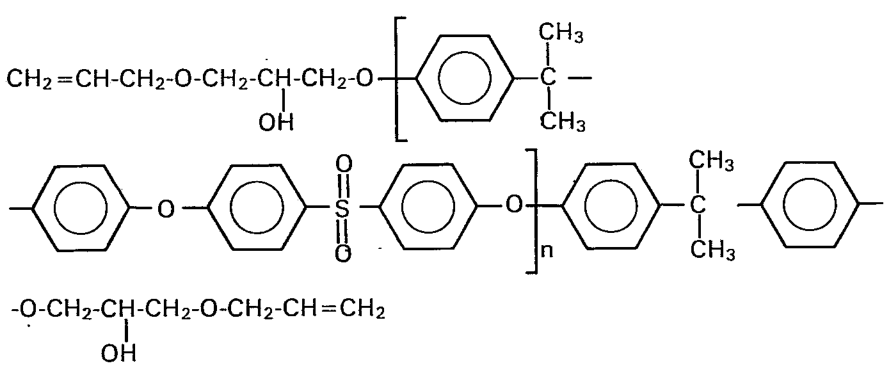

(X)

worin n eine ganze Zahl $\geq$ 1 bedeutet.

8. Polyphenolderivat nach Anspruch 6, dadurch gekennzeichnet, dass es der Formel entspricht:

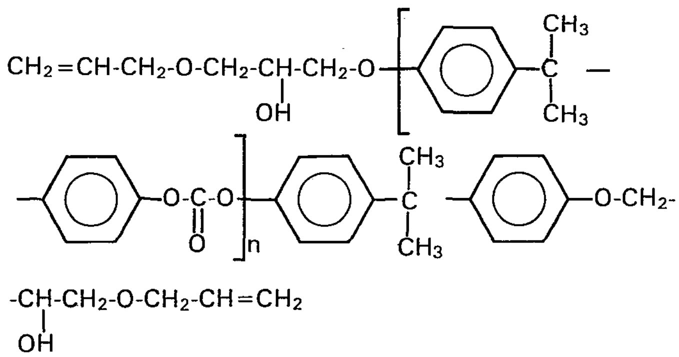

(XII)

worin n eine ganze Zahl ≥ 1 bedeutet.

9. Polyphenolderivat nach Anspruch 7, dadurch gekennzeichnet, dass n = 4 ist.

10. Polyphenolderivat, dadurch gekennzeichnet, dass es der Formel entspricht:

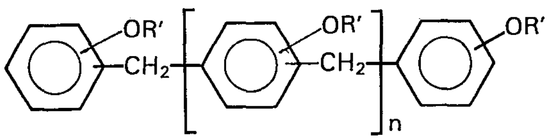

(XIV)

worin R' 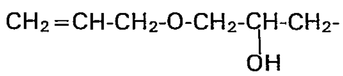

und n eine ganze Zahl ≥ 1 bedeuten.

11. Sequenzcopolymeres aus einem Polyphenolderivat, hergestellt durch Umsetzung eines Polyphenols mit 1-Allyloxy-2,3-epoxy-propan, und einem Polysiloxan der Formel

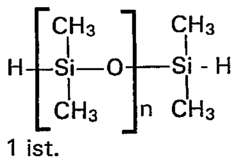

(XV)

worin n ≥ 1 ist.

12. Copolymeres nach Anspruch 11, dadurch gekennzeichnet, dass es sich bei dem Polyphenolderivat um 2,2-(Bis-3-allyloxy-2-hydroxy-4-propanoxy-phenyl)propan handelt.

13. Copolymeres nach Anspruch 11, dadurch gekennzeichnet, dass das Polyphenolderivat der Formel entspricht:

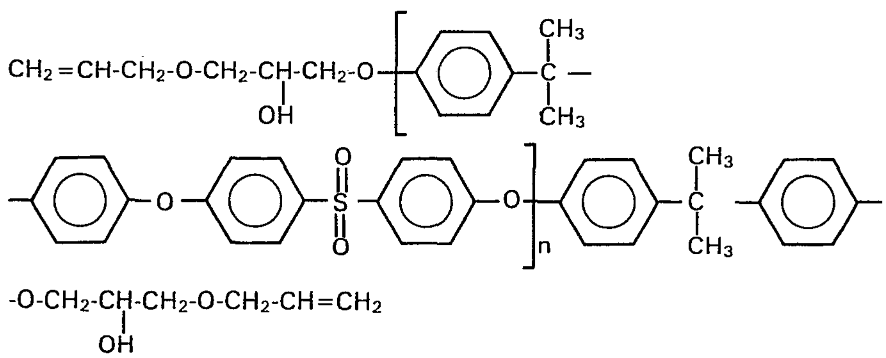

(X)

worin n eine ganze Zahl ≥ 1 bedeutet.

**Claims**

1. Process for the preparation of a polyphenol derivative comprising at least two reactive functions of different types at each of its ends, characterized in that it comprises reacting 1-allyloxy-2,3-epoxy-propane of the formula:

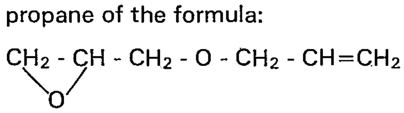

with a polyphenol selected from the following compounds:

- the polyphenol having the formula:

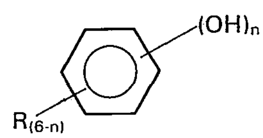

(I)

in which R represents a hydrogen atom, a halogen

atom, a $C_1$ to $C_5$ alkyl group or a $C_1$ to $C_5$ alkoxy group, and n is a whole number from 3 to 6;

- the polyphenol having the formula:

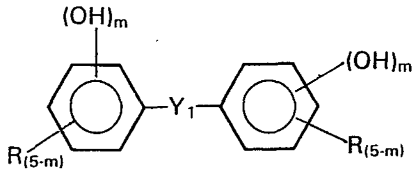

(II)

in which R represents a hydrogen atom, a halogen atom, a $C_1$ to $C_5$ alkyl group or a $C_1$ to $C_5$ alkoxy group, $Y_1$ represents

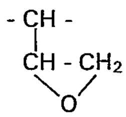 or the group 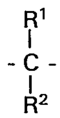

or $R^1$ and $R^2$, which can be the same or different, represent a halogen atom, a $C_1$ to $C_4$ alkoxy group, a $C_3$ to $C_6$ cycloalkyl group or the group 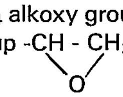 and

m is a whole numer from 1 to 5;

- the polyphenol having the formula

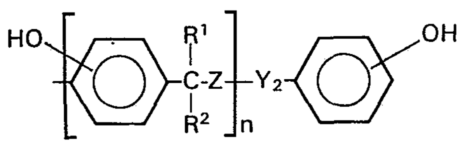

(III)

in which $R^1$ and $R^2$, which can be the same or different, each represents hydrogen, a halogen atom, a $C_1$ to $C_4$ alkyl group, a $C_1$ to $C_4$ alkoxy group, a $C_3$ to $C_6$ cycloalkyl group, or a group of the formula 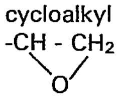

Z represents the group:

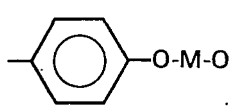

(A)

in which M represents -CO or

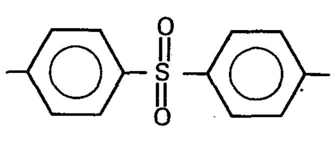

(B)

$Y_2$ represents the group

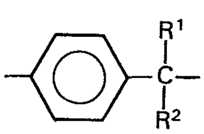

(C)

or the group

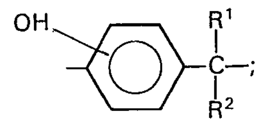

(D)

n is a whole number greater than or equal to 1;

- the polyphenol having the formula:

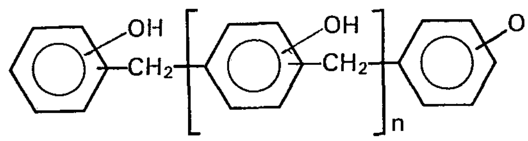

(IV)

in which n is a whole number equal to or greater than 1; and

- polyphenols selected from the group comprising 1,1-bis-(4-hydroxyphenylcyclohexane) (bis-phenol C) and fluoroglucinol,

in the presence of a basic catalyst comprising either a tertiary amine or an alkaline hydroxide.

2. Process according to claim 1, characterized in that the tertiary amine is N,N-dimethyldodecylamine or triethanolamine.

3. Process according to either of claims 1 and 2, characterized in that the reaction is carried out in the absence of a solvent at a temperature sufficient to produce solution of the polyphenol in 1-allyloxy-2,3--epoxy-propane.

4. Polyphenol derivative, characterized in that it has the formula:

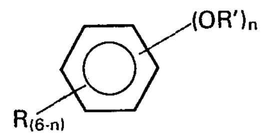

(V)

in which R represents a hydrogen atom, a halogen atom, a $C_1$ to $C_5$ alkyl group or a $C_1$ to $C_5$ alkoxy group, n represents a whole number from 3 to 6, and R′ represents a group of the formula:

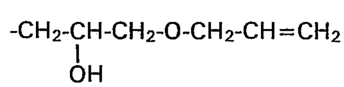

5. Polyphenol derivative, characterized in that it has the following formula:

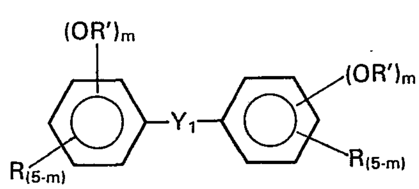

(VI)

in which R′ represents:

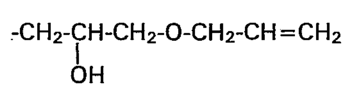

R represents a hydrogen atom, a halogen atom, a $C_1$ to $C_5$ alkyl group or a $C_1$ to $C_5$ alkoxy group;

$Y_1$ represents

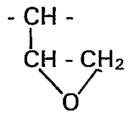

or the group

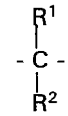

in which $R^1$ and $R^2$, which may be the same or diffrent, each represents a halogen atom, a $C_1$ to $C_4$ alkoxy group, a $C_3$ to $C_6$ cycloalkyl group, or the group 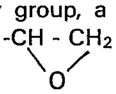 and

m is a whole number from 1 to 5.

6. Polyphenol derivative, characterized in that it has the following formula

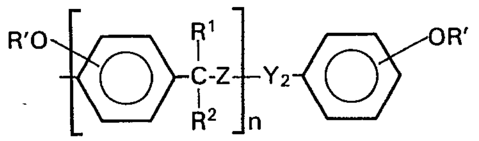

(VII

in which R′ represents:

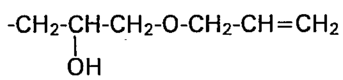

$R^1$ and $R^2$, which can be the same or diffrent, represent a hydrogen atom, a halogen atom, a $C_1$ to $C_4$ alkyl group, a $C_1$ to $C_4$ alkoxy group, a $C_3$ to $C_6$ cycloalkyl group, or the group 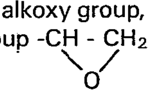

Z represents the group

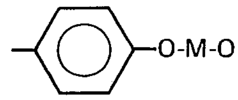

(A)

in which M represents CO or

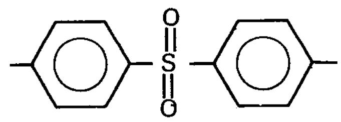

(B)

$Y_2$ represents the group:

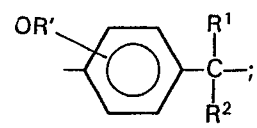

(C)

or the group

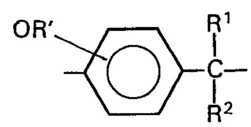

(D)

and n is a whole number greater than or equal to 1.

7. Polyphenol derivative according to claim 6, characterized in that it has the formula:

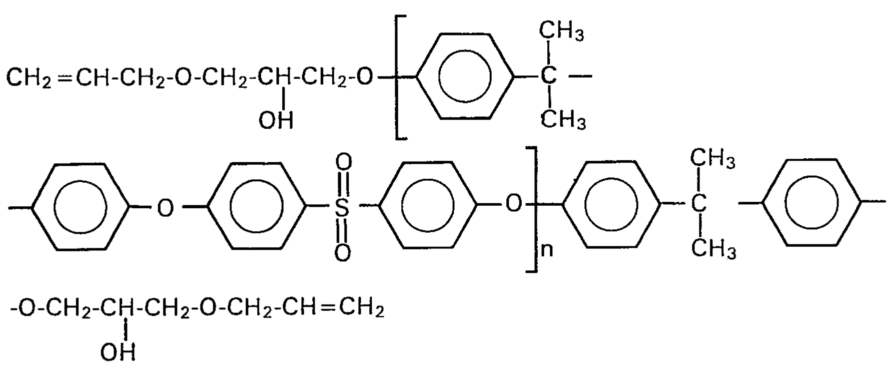

(X)

in which n is a number equal to or greater than 1.

8. Polyphenol derivative according to claim 6, characterized in that it has the formula:

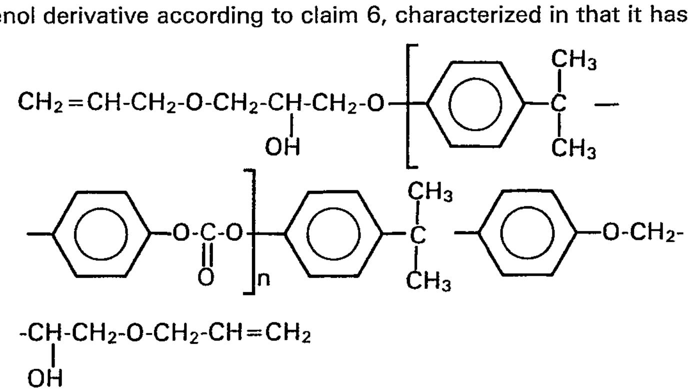

(XII)

in which n is a whole number greater than or equal to 1.

9. Polyphenol derivative according to claim 7, characterized in that n is equal to 4.

10. Polyphenol derivative, characterized in that it has the formula:

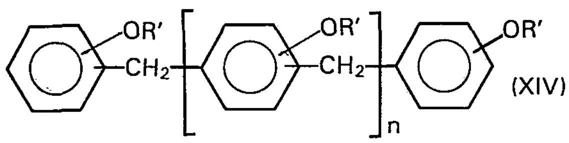

(XIV)

which R′ represents

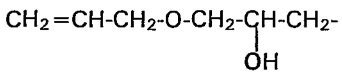

and n is a whole number greater than or equal to 1.

11. Block copolymer of a polyphenol derivative obtained by reaction of a polyphenol with 1-allyloxy-2,3-epoxypropane, and a polysiloxane of the formula:

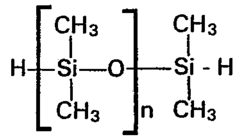

(XV)

in which n is greater than or equal to 1.

12. Copolymer according to claim 11, characterized in that the polyphenol derivative is 2,2-bis-[4-(3-allyloxy-2-hydroxy-propanoxy)-phenyl]-propane.

13. Copolymer according to claim 11, characterized in that the polyphenol derivative has the formula:

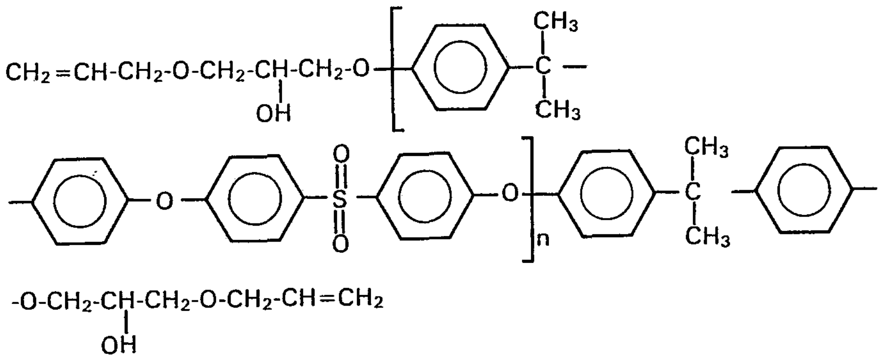

(X)

in which n is a whole number greater than or equal to 1.